# EUROPÄISCHE PATENTANMELDUNG

(11) **EP 2 384 783 A1**
(43) Veröffentlichungstag der Anmeldung: **09.11.2011**
(21) Anmeldenummer: 11164621.2
(22) Anmeldetag: 03.05.2011
(51) Int. Cl.: A61M 25/00, A61M 25/06, A61M 31/00

(54) **Vorgeformter Herzkatheter zur Durchführung einer Koronarangiographie**

(30) Priorität: 03.05.2010 DE 102010019243
(71) Anmelder: GMI German Medical Innovation GmbH, 53115 Bonn (DE)
(72) Erfinder: Lewalter, Thorsten, 53127, Bonn (DE)
(74) Vertreter: Kietzmann, Lutz

(57) **Zusammenfassung**

Die Erfindung betrifft einen vorgeformten Herzkatheter zur Durchführung einer Koronarangiographie mit einem durchgängigen Hohllumen (1) zum Injizieren einer Röntgenkontrastflüssigkeit in Herzkranzgefäße, welches abschnittsweise einstückig zusammengesetzt aus einem geraden und steifen Schaftabschnitt (3) besteht, dem sich endseitig über eine proximale Krümmung (4) ein distaler Brückenabschnitt (5) anschließt, welcher über eine distale Krümmung (6) in einen distalen Spitzenabschnitt (7) einmündet, wobei das Hohllumen (1) zur unmittelbar aufeinanderfolgenden Anwendung in der rechten Herzarterie (10) und der linken Herzarterie (11) konfigurierbar ist, indem dieses eine federnde proximale Krümmung (4) aufweist, die durch eine mit dem Hohllumen (1) bis mindestens zum distalen Brückenabschnitt (5) verlaufenden Schleuse (2) derart vorspannbar ist, dass das Hohllumen (1) die Konfiguration zum Erreichen der rechten Herzarterie (10) annimmt, wobei ein Rückziehen der Schleuse (2) in den Bereich des Schaftabschnitts (3) eine Entspannung der vorgespannten proximalen Krümmung (4) auslöst, so dass das Hohllumen (1) die Konfiguration zum Erreichen der linken Herzarterie (11) annimmt.

## Beschreibung

Die vorliegende Erfindung bezieht sich auf einen vorgeformten Herzkatheter zur Durchführung einer Koronarangiographie, umfassend ein durchgängiges Hohllumen zum Injizieren einer Röntgenkontrastflüssigkeit in Herzkranzgefäße, welches abschnittsweise einstückig zusammengesetzt aus einem geraden und steifen Schaftabschnitt besteht, dem sich endseitig über eine proximale Krümmung ein distaler Brückenabschnitt anschließt, welcher über eine distale Krümmung in einen distalen spitzen Abschnitt einmündet.

Bei der Diagnose und Behandlung von Herzerkrankungen, welche durch einen Verschluss der Herzarterien bewirkt werden, kann ein Arzt verschiedene Tests und nicht-chirurgische Verfahren durchführen, in denen ein Katheter durch eine Arterie in den Arm oder das Bein und in die ausgewählte Herzarterie geführt wird. Sobald der Herzkatheter an Ort und Stelle ist, wird dieser dann zum Durchführen von diagnostischen Tests, wie einer Koronarangiographie oder auch für andere therapeutische Eingriffe verwendet.

Die Koronarangiographie ist eine Angiographie der Herzkranzgefäße und damit eine spezielle Form der Röntgenuntersuchung, bei der die Herzarterien abgebildet werden. Röntgenstrahlen machen eine Röntgenkontrastflüssigkeit sichtbar, welche zuvor in die Lumina der Herzkranzgefäße über den Herzkatheter injiziert werden.

Wogegen in früherer Zeit eine Oberschenkel- oder Oberarmkatheterisierung erfolgte, wird heutzutage meist ein Ansatz über die Speiche gewählt, wobei der Herzkatheter in die Aorta über die Speichenarterie im Handgelenk eingeführt wird. Die Speichenarterie und die Ellenarterie sind zwei kleine Arterien im Handgelenk, die durch den Hohlhandbogen kommunizieren. Vorteilhaft unterbindet eine Insertierung eines Herzkatheters in die Speichenartiere nicht den Blutfluss im unteren Arm oder der Hand, da das Blut fortfahren kann, durch die Ellenarterie und den Hohlhandbogen zu fließen. Dieser Ansatz erfordert ebenfalls eine verhältnismäßig kurze Erholungszeit von wenigen Minuten, um zu gewährleisten, dass eine Blutung aus der chirurgischen Wunde gestoppt ist. Somit ist ein Vorteil der Verwendung eines Ansatzes über die Speiche zur diagnostischen Kathetisierung und eines Eingriffs gegenüber Ansätzen einer Oberschenkel- oder Armkathetisierung die frühere Überführung des Patienten in eine ambulante Pflege. Zur optimalen Verwendung aus der Speichenarterie für die Durchführung einer Koronarangiographie existieren vorgeformte Herzkatheter.

Aus der EP 0 935 972 B1 gehen derartige vorgeformte spezielle Herzkatheter hervor. Der Herzkatheter umfasst ein durchgängiges Hohllumen für das Injizieren der Röntgenkontrastflüssigkeit, welcher zum perkutanten Ansatz über eine Speiche zur Kathetisierung vorgesehen ist. Hierbei werden zwei verschieden geformte Herzkatheter eingesetzt, um diese für die rechte bzw. linke Herzarterie zu verwenden. Der Herzkatheter für die rechte Herzarterie umfasst ein gestrecktes röhrenförmiges Hohllumen mit einem speziellen distalen Endbereich. Der distale Endbereich, der distal vom Körperbereich angeordnet ist, umfasst einen gekrümmten Bereich und einen verhältnismäßig geraden Brückenbereich dazwischen sowie einen spitzen Bereich, welcher das distale Ende des Herzkatheters bildet. Spezielle Winkelbereiche für Krümmungen und spezielle Abstände dazwischen stellen sicher, dass sich der Herzkatheter durch die Speiche oder Oberarmarterie in die aufsteigende Aorta einsetzen lässt, wobei der gekrümmte Bereich gegen die Medialwand der aufsteigenden Aorta positioniert ist, und wobei der Winkel des gekrümmten Bereiches ausreichend ist, um den Brückenbereich sowie den Spitzenbereich in Richtung auf die Lateralwand der Aorta zu lenken. Weiterhin ist auch der Winkel eines zweiten gekrümmten Bereiches ausreichend, um den Spitzenbereich in Richtung auf die rechte Herzarterie zum Intubieren in das Ostium der Herzarterie zu lenken. Hierzu besitzt der Herzkatheter eine im Wesentlichen langgestreckte Konfiguration.

Im Gegensatz hierzu besitzt der anderer Herzkatheter zum Erreichen der linken Herzarterie eine deutlich ausgeprägte Rückkrümmung des distalen Endbereichs, welche eingeleitet wird über einen ersten gekrümmten Bereich, der distal vom Körperbereich angeordnet ist und in einen deutlich mit einem Winkel zwischen 135° bis 155° gekrümmten zweiten Bereich, dem ein Verhältnismäßig gerader Spitzenbereich folgt. Während der erste gekrümmte Bereich einen Winkel zwischen 20 bis 40° einnimmt, nimmt der zweite gekrümmte Bereich einen Winkel zwischen 135 bis 155° in die entgegengesetzte Richtung ein. Mit dieser Konfiguration wird der erste gekrümmte Bereich gegen die Medialwand der aufsteigenden Aorta positioniert, dessen Winkel ausreichend ist, um den geraden Spitzenbereich in Richtung auf die Lateralwand der Aorta zu lenken. Weiterhin wird der zweite gekrümmte Bereich gegen die Lateralwand positioniert, dessen Winkel ausreichend ist, um den Spitzenbereich in Richtung auf die linke Herzarterie zur Intubation der linken Herzarterie zu richten.

Zwar sind die beiden verschiedenen Herzkatheter optimal an ihren jeweiligen Verwendungszweck angepasst, jedoch erfordert ein Injizieren einer Röntgenkontrastflüssigkeit in die linke und in die rechte Herzarterie einen Katheterwechsel, welcher meist über Drahttechnik durchgeführt wird. Hieraus resultiert der Nachteil, dass für den Katheterwechsel das Einlegen des Drahtes in die Aorta erforderlich wird, worüber der einzuwechselnde Herzkatheter vorgeführt wird. Diese Tätigkeit erweist sich als recht zeitaufwendig und birgt die potenzielle Gefahr einer Gefäßverletzung in sich. Ferner müssen beide Varianten von Herzkathetern bereitgestellt werden, was neben dem erforderlichen Aufwand für die Lagerhaltung auch zusätzlichen Zeitaufwand zur Vorbereitung beider Herzkatheter durch jeweiliges Auspacken, Durchspülen und dergleichen verursacht.

In der US 5,322,509 wird zur Lösung dieser Problematik daher ein einziger, universell geformter Herzkatheter vorgeschlagen, der für die Intubation der linken und der rechten Herzarterie konzipiert ist.

Zu diesem Zweck ist dieser universelle Herzkatheter im distalen Endbereich mit einer vorgeformten Doppelkurve versehen, welche spezielle Winkel- und Längenmaße aufweist, die einen im Wesentlichen C-förmige Kontur ergeben. Der so gestaltete distale Endbereich des Herzkatheters ist aus einem Material gefertigt, welches eine recht flexible Formänderung gestattet.

Der universelle Herzkatheter lässt sich durch seine spezielle Formgebung zunächst derart durch die Aorta einführen, dass durch Vorschieben die rechte Herzarterie zu erreichen ist. Soll nun auch die linke Herzarterie ohne Katheterwechsel erreicht werden, wird der distale Endbereich des Herzkatheters durch Verdrehen nach vorherigem kurzen Zurückziehen um 180° gewendet, so dass der Spitzenbereich nun auf die linke Herzarterie zielt.

Zwar lässt sich durch diesen universellen Herzkatheter ein Katheterwechsel vermeiden, allerdings müssen je nach den jeweiligen anatomischen Verhältnissen auch Kompromisse hinsichtlich der Zielgenauigkeit, insbesondere zum Erreichen der linken Herzarterie nach dem Drehen des distalen Endbereichs des Herzkatheters hingenommen werden, was den Eingriff nachteilhaft verlängern könnte. Des Weiteren wird die Konfigurationsänderung dieses vorgeformten Herzkatheters im Zusammenwirken mit der aortalen Wand als passives Widerlager für den Herzkatheter bewirkt, was neben einer Beeinflussung der Handhabbarkeit auch zu einer örtlichen Verletzungsgefahr führen kann.

Es ist daher die Aufgabe der vorliegenden Erfindung einen vorgeformten Herzkatheter zur Durchführung einer Koronarangiograpnie zu schaffen, mit dem sich schnell und ohne Katheterwechsel zielgenau sowohl die linke als auch die rechte Herzarterie erreichen lässt.

Die Erfindung schließt die technische Lehre ein, dass das Hohllumen des Herzkatheters zur unmittelbar aufeinanderfolgenden Anwendung in der rechten Herzarterie und der linken Herzarterie derart konfigurierbar ist, dass dieses eine federnde proximale Krümmung aufweist, die durch eine mit dem Hohllumen bis mindestens zum distalen Brückenabschnitt verlaufenden Schleuse derart vorspannbar ist, dass das Hohllumen die Konfiguration zum Erreichen der rechten Herzarterie annimmt, wobei ein Rückziehen der Schleuse in den Bereich des Schaftabschnitts eine Entspannung der vorgespannten proximalen Krümmung auslöst, so dass das Hohllumen anschließend die Konfiguration zum Erreichen der linken Herzarterie annimmt.

Mit anderen Worten wird ausgehend von einer Kurvenform, die der Konfiguration eines Herzkatheters zur Darstellung der rechten Herzarterie durch Rückzug der Schleuse eine federnd vorgespannte proximale Krümmung freigesetzt, woraufhin der Herzkatheter eine zweifach gekrümmte Form (sogenannte Judkins-Konfiguration) annimmt, welche zur Intubation der linken Herzarterie geeignet ist.

Der Vorteil der erfindungsgemäßen Lösung beruht auf einer Konfigurationsänderung, bei welcher die erforderliche zweifache Krümmung zunächst in einer Krümmung zusammengeführt wird. Durch das Vorschieben und Rückziehen der Schleuse kann der Konfigurationswechsel äußerst präzise erfolgen, so dass der erfindungsgegenständlich vorgeformte Herzkatheter sich schnell und flexibel an unterschiedliche anatomische Gegebenheiten anpassen lässt. Auf eine Einbeziehung von Gefäßwandungen zur Konfigurationsänderung kann dabei vollständig verzichtet werden. Des Weiteren entfällt natürlich ein Katheterwechsel, so dass die erfindungsgemäße Lösung zur Senkung des Herstellungs- und Bereitstellungsaufwandes beiträgt.

Gemäß einer bevorzugten Ausführungsform der erfindungsgemäßen Lösung wird vorgeschlagen, dass die Schleuse das Hohllumen außenrohrförmig umgibt, um die Winkeländerung der proximalen Krümmung herbeizuführen. Alternativ hierzu ist es auch denkbar, die Schleuse innerhalb des Hohllumens in Form eines Drahtes oder eingebettet in deren Wandung unterzubringen. Die bevorzugte Ausführungsform der äußeren Schleuse hat jedoch den Vorteil, dass sich hierdurch eine präzise Konfigurationsänderung der federnden proximalen Krümmung unter minimalem Kraftaufwand erreichen lässt. Außerdem lässt sich eine das Hohllumen außenrohrförmig umgebende Schleuse fertigungstechnisch einfach realisieren.

Vorzugsweise besteht die rohrförmige Schleuse aus einem mittelweichen Kunststoffmaterial, insbesondere einem Polyäthylen- oder Silikonkunststoff, mit einer Wandstärke von 0,5 bis 1,5 mm. Trotz relativ geringer Wandstärke lässt sich auf diese Weise eine zuverlässige Schleusenfunktion für das innenliegende Hohllumen erzeugen.

Das mit der äußeren Schleuse zusammenwirkende ebenfalls rohrförmige Hohllumen zum Indizieren der Röntgenkonstrastflüssigkeit besteht vorzugsweise aus einem relativ weichen Kunststoffmaterial, insbesondere einem Polyäthylen- oder Silikonkunststoff, mit einer Wandstärke von 0,2 bis 1 mm. Bei diesen Materialparametern lässt sich insbesondere auch die federnde proximale Krümmung hinsichtlich der erforderlichen Elastizität zuverlässig gestalten. Es können im Rahmen dieser Maßangaben vorzugsweise Herzkatheter der Größen 5F oder 6F hergestellt werden. Analog zu diesen speziellen Maßverhältnissen ist es natürlich auch möglich, andere gängige Größen zu verwirklichen.

Hinsichtlich einer bevorzugten Ausführungsform des Herzkatheters weist das Hohllumen einen Außendurchmesser im Bereich zwischen 2 bis 5 mm auf, welches von einer Schleuse mit Außendurchmesser im Bereich zwischen 3 bis 6 mm überzogen ist, um bei möglichst geringen geometrischen Abmessungen die erfindungsgemäße Funktionsweise zuverlässig umsetzen zu können.

In Anpassung auf die menschliche Anatomie der Aorta sowie der hiermit verbundenen rechten Herzarterie wird vorgeschlagen, das Hohllumen im vorgespannten Zustand bei geschlossener Schleuse zum Erreichen der rechten Herzarterie mit einer proximale Krümmung mit einem Winkel α₁ im Bereich zwischen 0° bis 30° zu versehen. Die proximale Krümmung fällt somit recht flach aus, so dass der Herzkatheter einen eher im Wesentlichen langgestreckten Endbereich erhält. Dagegen weist das Hohllumen im entspannten Zustand bei geöffneter Schleuse zum Erreichen der linken Herzarterie vorzugsweise eine proximale Krümmung mit einem Winkel α₂ im Bereich zwischen 130° bis 170° auf. Durch die Schleuse lässt sich die proximale Krümmung des erfindungsgemäßen Herzkatheters also zwischen den beiden Winkeln α₁ und α₂ in einfacher Weise variieren.

Im Übrigen wird vorgeschlagen, dass das Hohllumen mit einer distalen Krümmung ausgestattet ist, die sowohl im vorgespannten als auch im entspannten Zustand zum Erreichen der linken und der rechten Herzarterie einen Winkel β₁ im Bereich zwischen 90° und 110° besitzt. Diese distale Krümmung kann also für die linke als auch für die rechte Herzarterie ohne Beeinflussung der Krümmung durch die Schleuse genutzt werden.

Um eine möglichst ideale Anpassung für beide Konfigurationen des erfindungsgemäßen Herzkatheters zu schaffen, wird weiterhin vorgeschlagen, den distalen Brückenabschnitt, der zwischen den beiden Krümmungen des Endbereichs liegt, mit einer Länge zwischen 70 und 100 mm auszustatten. Hierzu angepasst sollte der distale Spitzenabschnitt eine Länge zwischen 10 bis 20 mm aufweisen. Durch diese Maße und insbesondere durch die Maßkombination ist der erfindungsgemäß vorgeformtem Herzkatheter besonders einfach und zielgenau handhabbar.

Zur weiteren Erleichterung der Handhabung wird gemäß eines anderen Aspekts der Erfindung vorgeschlagen, dass der Schaftabschnitt an dem mit dem Brückenabschnitt gegenüberliegenden Ende mit einer - an sich bekannten - Handhabungsmechanik ausgestattet ist, welche allerdings sowohl zum axialen als auch zum rotierenden Bewegen des Hohllumens sowie zum relativen axialen Verschieben desselben gegenüber der Schleuse nutzbar ist.

Weitere, die Erfindung verbessernde Maßnahmen werden nachstehend gemeinsam mit der Beschreibung eines bevorzugten Ausführungsbeispiels der Erfindung anhand der Figuren näher dargestellt. Es zeigt:
- Figur 1: eine schematische Seitenansicht eines vorgeformtem Herzkatheters in der für die rechte Herzarterie bestimmten Konfiguration,
- Figur 2: eine schematische Seitenansicht eines vorgeformtem Herzkatheters in der für die linke Herzarterie bestimmten Konfiguration.
- Figur 3: eine schematische Seitenansicht des Zusammenwirkens des Herzkatheters nach Figur 1 mit der rechte Herzarterie, und
- Figur 4: eine schematische Seitenansicht des Zusammenwirkens des Herzkatheters nach Figur 2 mit der linke Herzarterie.

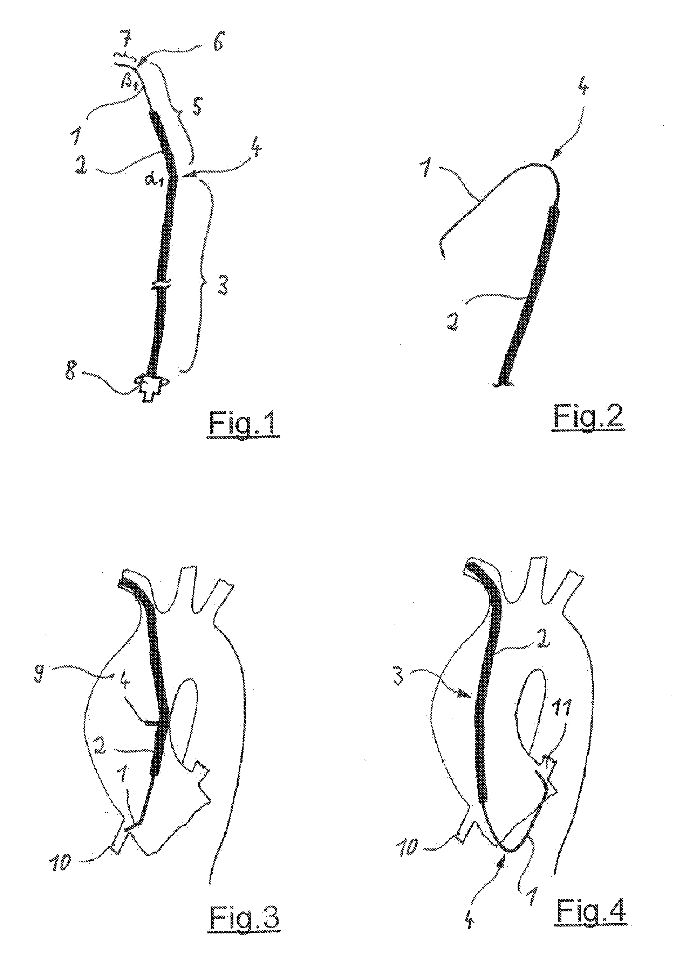

Gemäß Figur 1 weist ein vorgeformter Herzkatheter ein durchgängiges Hohllumen 1 zum Indizieren einer Röntgenkontrastflüssigkeit auf, das von einer rohrförmigen Schleuse 2 zur Konfigurationsänderung durch axiales Verschieben gegenüber dem Hohllumen 1 umgeben ist.

Das Hohllumen 1 ist durchgängig gestaltet und aus mehreren einzelnen Abschnitten zusammengesetzt, die jeweils spezielle Funktionen erfüllen. Ein gerader und steifer Schaftabschnitt 3 geht zum distalen Endbereich des Herzkatheters hin in eine proximale Krümmung 4 über, die hier recht flach ausfallend einen Winkel α₁ von ca. 20° aufweist. In dieser Konfiguration ist die Schleuse 2 vollständig über die proximale Krümmung 4 des Hohllumens 1 geschoben, um diesem die gestreckte Form zu verleihen.

Der hier flachen proximalen Krümmung 4 schließt sich ein distaler Brückenabschnitt 5 einer Länge von ca. 90 mm an, welcher über eine distale Krümmung 6 mit einem Winkel β₁ von ca. 100° in einen distalen Spitzenabschnitt 7 einmündet, der in diesem Ausführungsbeispiel ca. 15 mm lang ist. Die Längenmaße sind jeweils vom Scheitelpunkt der vorstehend erwähnten Krümmungen aus bemessen.

An dem gegenüberliegenden Ende des distalen Endbereichs des Herzkatheters weist dieser eine - hier nur schematisch angedeutete - Handhabungsmechanik 8 zum axialen und rotierenden Bewegen des Hohllumens 1 sowie zum relativen axialen Verschieben desselben gegenüber der Schleuse 2 auf.

Gemäß Figur 2 befindet sich der vorgeformte Herzkatheter in einer anderen Konfiguration, die zum Erreichen der linken Herzarterie vorgesehen ist. Hierbei ist durch Zurückziehen der Schleuse 2 vom Hohllumen 1 die federnde proximale Krümmung 4 freigelegt, so dass diese mangels Zwangsausrichtung selbstständig von der Konfiguration zum Erreichen der rechten Herzarterie -wie vorstehend beschrieben - in die Konfiguration zum Erreichen der linken Herzarterie wechselt, in welcher die federnde proximale Krümmung 4 in den entspannten Zustand gelangt.

Wie Figur 3 illustriert, befindet sich der vorgeformte Herzkatherter innerhalb einer Aorta 9 kurz vor Erreichen der rechten Herzarterie 10. In dieser Konfiguration ist die Schleuse 2 über die federnde proximale Krümmung 4 geschoben, um das Hohllumen 1 im distalen Endbereich in die gewünschte gestreckte Form zu bringen.

Durch das in Figur 4 dargestellte Zurückziehen der Schleuse 2 vom Hohllumen 1 in den Schaftabschnitt 3 tritt eine Entspannung der vorgespannten proximalen Krümmung 4 ein, so dass das Hohllumen 1 die Konfiguration zum Erreichen der linken Herzarterie 11 einnimmt.

Die Erfindung ist nicht beschränkt auf das vorstehend beschriebene bevorzugte Ausführungsbeispiel. Es sind vielmehr auch Änderungen hiervon denkbar, welche vom Schutzbereich der nachfolgenden Ansprüche mit umfassen. So ist es beispielsweise auch möglich, die vorstehend genannten Parameter für Winkel und Abschnittslängen des Herzkatheters je nach anatomischen Erfordernissen zu ändern.

### Bezugszeichenliste

- 1: Hohllumen
- 2: Schleuse
- 3: steifer Schaftabschnitt
- 4: proximale Krümmung
- 5: distaler Brückenabschnitt
- 6: distale Krümmung
- 7: distaler Spitzenabschnitt
- 8: Handhabungsmechanik
- 9: Aorta
- 10: rechte Herzarterie
- 11: linke Herzarterie

- α₁: erster proximaler Krümmungswinkel
- α₂: zweiter proximaler Krümmungswinkel
- β₁: distaler Krümmungswinkel

## Patentansprüche

1. Vorgeformter Herzkatheter zur Durchführung einer Koronarangiographie mit einem durchgängigen Hohllumen (1) zum Injizieren einer Röntgenkontrastflüssigkeit in Herzkranzgefäße, welches abschnittsweise einstückig zusammengesetzt aus einem geraden und steifen Schaftabschnitt (3) besteht, dem sich endseitig über eine proximale Krümmung (4) ein distaler Brückenabschnitt (5) anschließt, welcher über eine distale Krümmung (6) in einen distalen Spitzenabschnitt (7) einmündet,
**dadurch gekennzeichnet, dass** das Hohllumen (1) zur unmittelbar aufeinanderfolgenden Anwendung in der rechten Herzarterie (10) und der linken Herzarterie (11) konfigurierbar ist, indem dieses eine federnde proximale Krümmung (4) aufweist, die durch eine mit dem Hohllumen (1) bis mindestens zum distalen Brückenabschnitt (5) verlaufenden Schleuse (2) derart vorspannbar ist, dass das Hohllumen (1) die Konfiguration zum Erreichen der rechten Herzarterie (10) annimmt, wobei ein Rückziehen der Schle5use (2) in den Bereich des Schaftabschnitts (3) eine Entspannung der vorgespannten proximalen Krümmung (4) auslöst, so dass das Hohllumen (1) die Konfiguration zum Erreichen der linken Herzarterie (11) annimmt.

2. Vorgeformter Herzkatheter nach Anspruch 1,
**dadurch gekennzeichnet, dass** die Schleuse (2) das Hohllumen (1) außen rohrförmig umgibt, um die Winkeländerung der proximalen Krümmung (4) herbeizuführen.

3. Vorgeformter Herzkatheter nach Anspruch 2,
**dadurch gekennzeichnet, dass** die rohrförmige Schleuse (2) je nach Kathetergröße aus einem Kunststoffmaterial mit einer Wandstärke von 0,5 bis 1,5 Millimetern besteht.

4. Vorgeformter Herzkatheter nach Anspruch 2,
**dadurch gekennzeichnet, dass** das mit der äußeren Schleuse (2) zusammenwirkende ebenfalls rohrförmige Hohllumen (1) aus einem weichen Kunststoffmaterial mit einer Wandstärke von 0,2 bis 1 Millimeter besteht.

5. Vorgeformter Herzkatheter nach Anspruch 2,
**dadurch gekennzeichnet, dass** die Schleuse (2) einen Außendurchmesser im Bereich zwischen 3 bis 6 Millimetern aufweist, in welcher das Hohllumen (1) mit einem Außendurchmesser im Bereich zwischen 2 und 5 Millimetern verläuft.

6. Vorgeformter Herzkatheter nach Anspruch 1,
**dadurch gekennzeichnet, dass** das Hohllumen (1) im vorgespannten Zustand bei geschlossener Schleuse (2) zum Erreichen der rechten Herzarterie (10) eine proximale Krümmung (4) mit einem Winkel α₁ im Bereich zwischen 0° und 30° aufweist.

7. Vorgeformter Herzkatheter nach Anspruch 1,
**dadurch gekennzeichnet, dass** das Hohllumen (1) im entspannten Zustand bei geöffneter Schleuse (2) zum Erreichen der linken Herzarterie (11) eine proximale Krümmung (4) mit einem Winkel α₂ im Bereich zwischen 130° bis 170° aufweist.

8. Vorgeformter Herzkatheter nach Anspruch 1,
**dadurch gekennzeichnet, dass** das Hohllumen (1) im vorgespannten sowie entspannten Zustand zum Erreichen der linken und rechten Herzarterie (10) eine distale Krümmung (6) mit einem Winkel β₁ im Bereich zwischen 90° und 110° aufweist.

9. Vorgeformter Herzkatheter nach Anspruch 1,
**dadurch gekennzeichnet, dass** der distale Brückenabschnitt (5) eine Länge zwischen 70 und 100 Millimetern aufweist.

10. Vorgeformter Herzkatheter nach Anspruch 1,
**dadurch gekennzeichnet, dass** der distale Spitzenabschnitt (7) eine Länge zwischen 10 bis 20 Millimetern aufweist.

11. Vorgeformter Herzkatheter nach einem der vorstehenden Ansprüche,
**dadurch gekennzeichnet, dass** der Schaftabschnitt (3) an dem dem Brückenabschnitt (5) gegenüberliegenden Ende mit einer Handhabungsmechanik (8) zum axialen und rotierenden Bewegen des Hohllumens (1) sowie zum relativen axialen Verschieben desselben gegenüber der Schleuse (2) verbindbar ist.
